# FASCICULE DE BREVET EUROPEEN

(11) **EP 1 189 933 B1**
(45) Date de publication et mention de la délivrance du brevet: **09.04.2003**
(21) Numéro de dépôt: 00942169.4
(22) Date de dépôt: 08.06.2000
(51) Int. Cl.: C07K 7/56, A61K 38/12, A61P 31/10

(54) **DERIVES DE L'ECHINOCANDINE, LEUR PROCEDE DE PREPARATION ET LEUR APPLICATION COMME ANTIFONGIQUES**
ECHINOCANDINDERIVATE, DEREN HERSTELLUNG UND DEREN VERWENDUNG ALS FUNGIZID
ECHINOCANDIN DERIVATIVES, METHOD FOR PREPARING THE SAME AND APPLICATION AS ANTIFUNGAL AGENTS

(30) Priorité: 09.06.1999 FR 9907251
(43) Date de publication de la demande: 27.03.2002
(73) Titulaire: Aventis Pharma S.A., 92160 Antony (FR)
(72) Inventeur: FAUVEAU, Patrick, F-93190 Livry Gargan (FR); HAWSER, Stephen, I-37020 Arbizzano di Valpolicella (IT); LEBOURG, Gilles, F-93220 Gagny (FR); SCHIO, Laurent, F-93140 Bondy (FR)
(74) Mandataire: Rousseau, Pierrick Edouard
(86) Numéro de dépôt international: FR0001568
(87) Numéro de publication internationale: WO00075177

(56) Documents cités:
- WO-A-96/13272
- WO-A-99/29716

## Description

La présente invention concerne de nouveaux dérivés de l'échinocandine, leur procédé de préparation et leur application comme antifongiques.

L'invention a pour objet, sous toutes les formes d'isomères possibles ainsi que leurs mélanges, les composés de formule (I) : 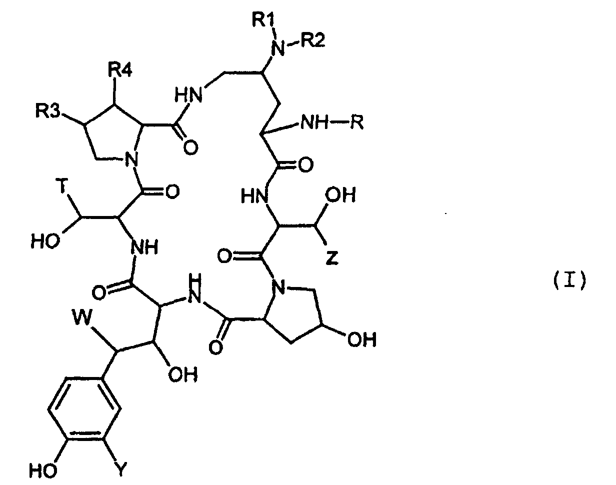 dans lesquels
ou bien R₁ représente un atome d'hydrogène ou un radical méthyle.
R₂ représente un radical cyclohexyle substitué par une amine, un radical CH₂CH₂NHCH₃, un radical CH₂CHCH₃NH₂, un radical 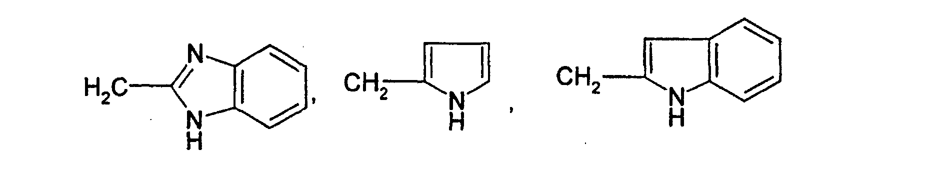 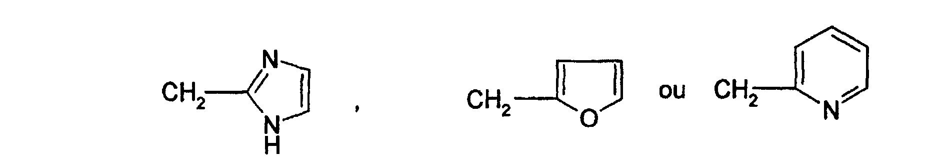 un radical CHCH₃CH₂NH₂, un radical-(CH₂)aOH, a représentant un nombre entier compris entre 1 et 8, un radical (CH₂)b-C≡N b représentant un nombre entier compris entre 1 et 8, un radical CHCH₃C₆H₅, un radical (CH₂)-C(CH₃)₂NHCOCF₃, un radical CHCH₃(CH₂)dOH, d représentant un nombre entier compris entre 1 et 8
ou bien R₁ et R₂ forment avec l'azote qui les porte un cycle à 3, 4 ou 5 carbones éventuellement substitué par une amine
R₃ représente un atome d'hydrogène, un radical méthyle ou hydroxyle
R₄ représente un atome d'hydrogène ou un radical hydroxyle
R représente une chaîne linéaire ou ramifiée ou cyclique renfermant jusqu'à 30 atomes de carbone, renfermant éventuellement, un ou plusieurs hétéroatomes, un ou plusieurs hétérocycles ou un radical acyle linéaire, ramifié ou cyclique renfermant jusqu'à 30 atomes de carbone renfermant éventuellement un ou plusieurs hétéroatomes et/ou un ou plusieurs hétérocycles,
T représente un atome d'hydrogène, un radical méthyle, un radical CH₂CONH₂, CH₂C≡N, un radical (CH₂)₂NH₂ ou (CH₂)₂Nalc⁺X⁻,
X étant un atome d'halogène et alc un radical alkyle renfermant jusqu'à 8 atomes de carbone,
Y représente un atome d'hydrogène, un radical hydroxyle ou un atome d'halogène ou un radical OSO₃H ou l'un des sels de ce radical,
W représente un atome d'hydrogène ou un radical OH,
Z représente un atome d'hydrogène ou un radical méthyle, ainsi que les sels d'addition avec les acides des produits de formule (I).

Parmi les sels d'addition avec les acides, on peut citer ceux formés avec les acides minéraux, tels que les acides chlorhydrique, bromhydrique, sulfurique ou phosphorique ou avec les acides organiques comme l'acide formique, acétique, trifluoroacétique, propionique, benzoïque, maléique, fumarique, succinique, tartrique, citrique, oxalique, glyoxylique, aspartique, alcanesulfoniques, tels que les acides méthane ou éthane sulfoniques, arylsulfoniques tels que les acides benzène ou paratoluènesulfoniques.

Parmi les composés préférés de l'invention, on peut citer tout particulièrement les composés de formule I dans lesquels T représente un atome d'hydrogène, ceux dans lesquels W représente un atome d'hydrogène, ceux dans lesquels Z représente un radical méthyle, ceux dans lesquels Y représente un atome d'hydrogène, ceux dans lesquels R₃ représente un radical méthyle, ceux dans lesquels R₄ représente un radical hydroxyle, et ceux dans lesquels R représente un radical

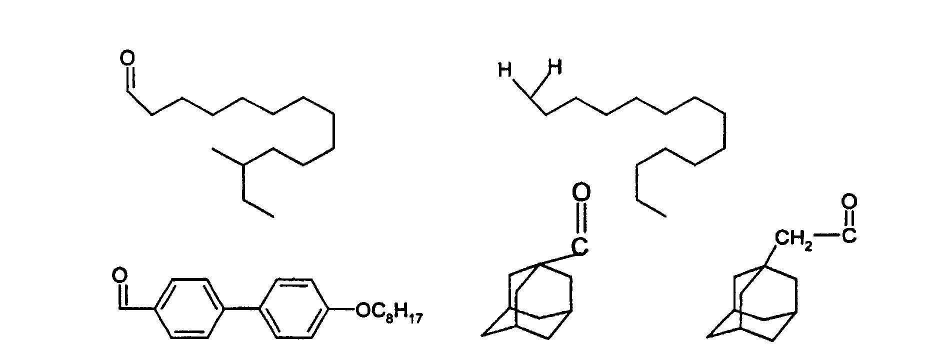

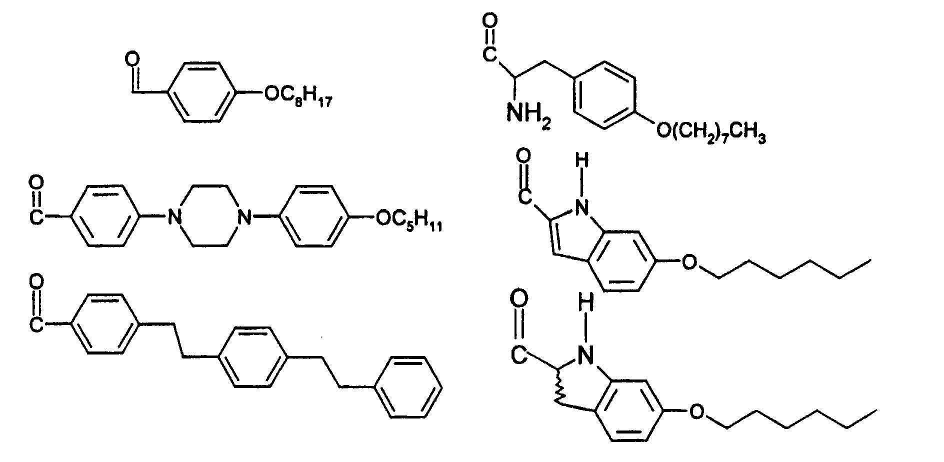

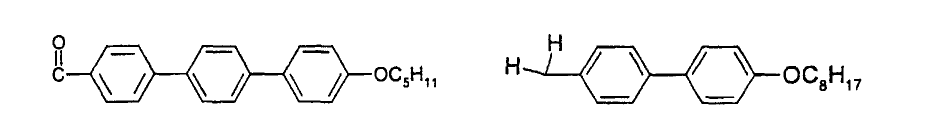

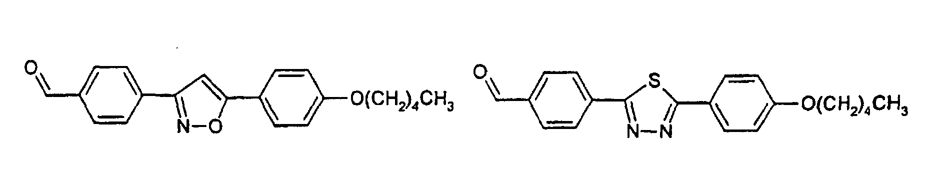

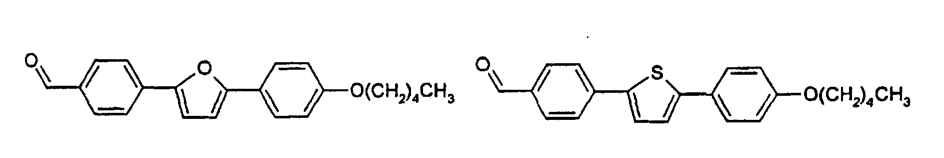

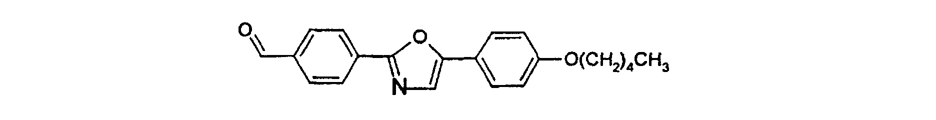

L'invention a tout spécialement pour objet les composés de formule I dans lesquels R représente une chaîne 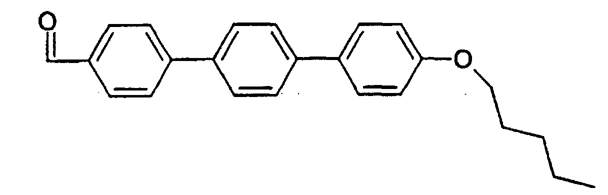 ou une chaîne

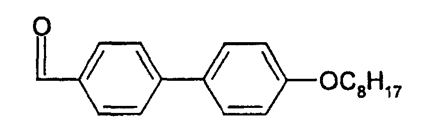

Parmi les composés préférés de l'invention, on peut citer tout particulièrement les composés de formule I dans lesquels R₁ est un atome d'hydrogène, ceux dans lesquels R₂ est un radical 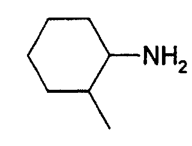 ceux dans lesquels R2 est un radical 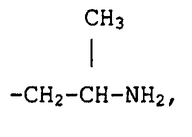 un radical 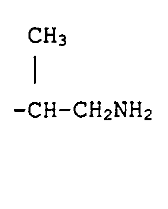 ou un radical 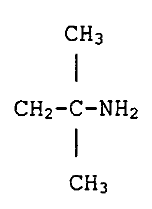 ou encore ceux dans lesquels R₂ est un radical

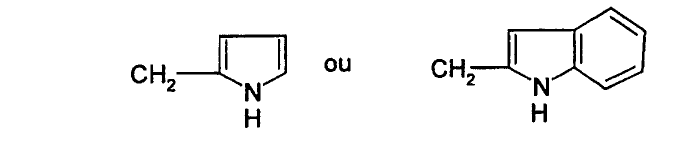

L'invention a tout spécialement pour objet les composés de formule (I), dont la préparation est donnée ci-après dans la partie expérimentale et notamment les produits des exemples 2 et 3.
Les composés de formule (I) présentent d'intéressantes propriétés antifongiques ; ils sont notamment actifs sur Candida albicans et autres Candida comme Candida glabrata, krusei, tropicalis, pseudotropicalis, parapsilosis et Aspergillus fumigatus, Aspergillus flavus, Cryptococcus neoformans.

Les composés de formule (I) peuvent être utilisés en tant que médicaments chez l'homme ou l'animal, pour lutter notamment contre les candidoses invasives de l'immunodéprimé, les candidoses digestives, urinaires, vaginales ou cutanées, les cryptococcoses, par exemple les cryptococcoses neuroméningées, pulmonaires ou cutanées, les aspergilloses bronchopulmonaires et pulmonaires et les aspergilloses invasives de l'immunodéprimé.
Les composés de l'invention peuvent être utilisés également dans la prévention des affections mycosiques chez les déprimés immunitaires congénitaux ou acquis.
Les composés de l'invention ne sont pas limités à une utilisation pharmaceutique, ils peuvent être également utilisés comme fongicides dans d'autres domaines que pharmaceutiques.

L'invention a donc pour objet à titre de composés antifongiques, les composés de formule (I) ainsi que leurs sels d'addition avec les acides.

L'invention a également pour objet les composés de formule (I), à titre de médicaments.

L'invention a tout particulièrement pour objet les compositions pharmaceutiques renfermant comme principe actif au moins un composé de formule (I) ou l'un de ses sels d'addition avec les acides pharmaceutiquement acceptables. Ces compositions peuvent être administrées par voie orale, rectale, parentérale ou par voie locale en application topique sur la peau et les muqueuses, mais les voies préférées sont les voies orale et parentérale.

Elles peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables, les pommades, les crèmes, les gels; elles sont préparées selon les méthodes usuelles. Le ou les principes actifs peuvent y être incorporés à des excipients habituellement employés dans ces compositions pharmaceuti ques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent également se présenter sous forme d'une poudre destinée à être dissoute extemporanément dans un véhicule approprié, par exemple de l'eau stérile apyrogène.

La dose administrée est variable selon l'affection traitée, le sujet en cause, la voie d'administration et le produit considéré. Elle peut être, par exemple, comprise entre 50 mg et 1 g par jour par voie orale ou parentérale, chez l'adulte pour les produits des exemples 2 et 3.

L'invention a également pour objet un procédé de préparation caractérisé en ce que l'on soumet un composé de formule (II) 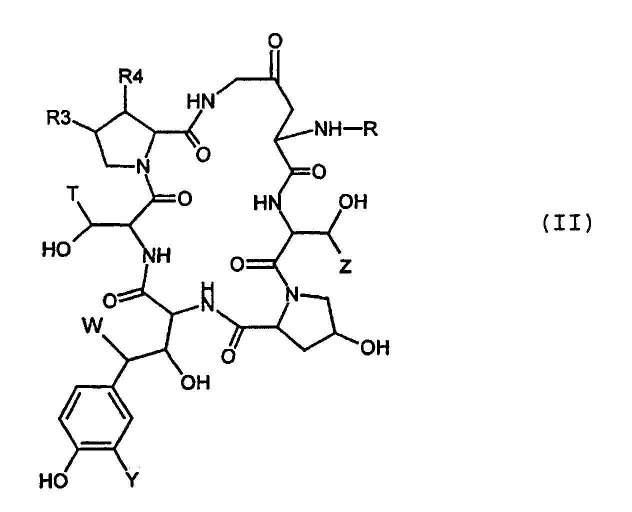 dans laquelle R, R₃, R₄, T, Y, W et Z conservent leur signification précédente, à l'action d'une amine ou d'un dérivé d'amine susceptible d'introduire
le radical 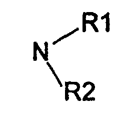 dans lequel R₁ et R₂ conservent leur signification précédente et si désiré à l'action d'un agent de réduction
et/ou d'un agent de fonctionnalisation de l'amine,
et/ou d'un acide pour former le sel du produit obtenu,
et/ou d'un agent de séparation des différents isomères obtenus,
et obtient ainsi le composé de formule (I) recherché.

Les composés de formule (II) décrits et revendiqués dans la demande de brevet WO 99 29716 peuvent être préparés selon un procédé caractérisé en ce que l'on soumet un composé de formule (III) 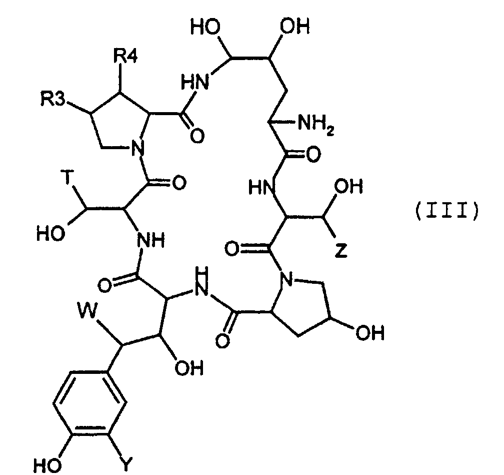 dans laquelle les différents substituants conservent leur signification précédente à l'action d'un agent capable de remplacer NH₂ par NHR, R conservant sa signification précédente pour obtenir le composé de formule (IV) 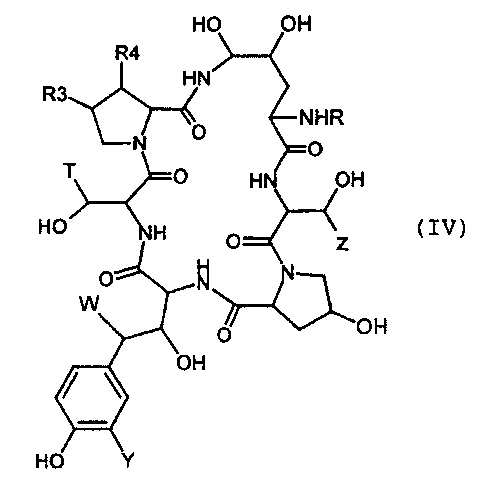 que l'on soumet à l'action de l'iodure de triméthylsilyle pour obtenir le composé de formule (II) correspondant

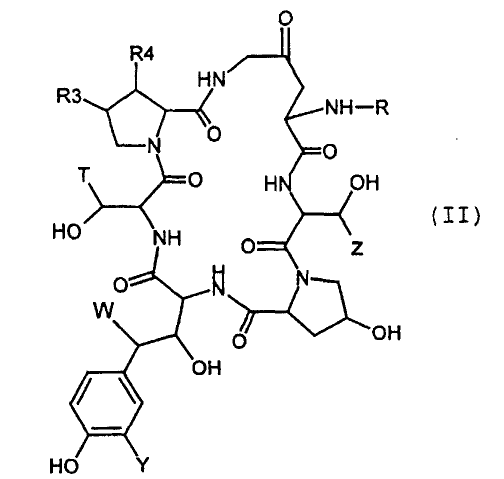

Les exemples suivants illustrent l'invention sans toutefois la limiter.

### Préparation 1 : "nucléus" de déoxymulundocandine

On dissout 2 g de déoxymulundocandine dans 20 ml de DMSO. On verse cette solution dans une suspension renfermant 120 g d'Actinoplanes utahensis FH2264 dans 870 ml d'un tampon KH2PO4, K2HPO (pH : 6.8). On maintient le mélange réactionnel sous agitation pendant 70 heures à 30°C. On filtre. On lave le mycelium avec le tampon de phosphate (pH : 6.8). On réunit les liquides de lavage et le filtrat. On chromatographie le produit obtenu sur une résine DIAION HP 20.et obtient un produit que l'on utilise tel quel ci-après.

### EXEMPLE 1 : Trifluoroacétate de 1-[4-[((2S)-2-amino-2-méthyléthyl)-amino]-N2-[[4'-(octyloxy)[1,1'-biphényl]-4-yl]-carbonyl]-L-ornithine]-4-[4-(4-hydroxyphényl)-L-threonine]5-L-serine-échinocandine B(isomère A et isomère B).

### Stade A : 1-[(4R,5R)-4,5-dihydroxy-N2-[[4'-(octyloxy)[1,1'-biphényl]-4-yl]-carbonyl]-L-ornithine]-4-[4-(4-hydroxyphényl)-L-thréonine]-5-L-sérine échinocandine B

### 1- Préparation de l'ester

On ajoute 632 mg de 2,3,4,5,6 pentafluorophénol et 695 mg de N,N'-dicyclohexylcarbodiimide à 1 g d'acide 4'-octyloxy-[1,1'-biphényl]4-carboxylique dans 22 ml de tétrahydrofurane, agite 22 heures à température ambiante, filtre, élimine les solvants sous pression réduite, reprend le résidu dans l'éther, agite à 35°C environ, filtre évapore le solvant , sèche et récupère 1,46 g de produit attendu, utilisé tel quel.

### 2- Couplage

On introduit 677 mg de " nucléus " de déoxymulundocandine obtenu à la préparation 1, dans 16 ml de DMF. On agite la solution obtenue pendant 5 minutes et ajoute 793 mg de 4'-(octyloxy)-[1,1'-biphényl]-4-carboxylate de pentafluorophényle obtenu ci-dessus. On maintient le mélange réactionnel sous agitation et atmosphère d'azote pendant 24 heures. On filtre et concentre. On reprend le résidu à l'éther, triture, maintient 25 minutes sous agitation, essore, lave à l'éther éthylique, chromatographie sur silice en éluant avec le mélange chlorure de méthylène, méthanol, eau (86/13/1) puis (80/20/1). On obtient ainsi le produit recherché. Rendement 73%.

### Stade B : 1-[N2-[[4'-(octyloxy)-[1,1'-biphényl]-4-yl] carbonyl]-4-oxo-L-ornithine]-4-[4-(4-hydroxyphényl)-L-thréonine]-5-L-serine-échinocandine B.

On ajoute 311 µl d'iodure de triméthylsilyle dans une suspension renfermant 809 mg de produit du stade A et 19 ml d'acétonitrile. On maintient le mélange réactionnel sous agitation pendant 15 minutes à 60°C et sous atmosphère d'azote. On verse le mélange dans une solution saturée en thiosulfate de sodium. On évapore et chromatographie sur silice le résidu obtenu, en éluant avec le mélange chlorure de méthylène-méthanol eau 86/13/1. On obtient le produit recherché. Rendement 55%.

### Stade C : Trifluoroacetate de 1-[4-[((2S)-2-amino-2-méthyléthyl)amino]-N2-[[4'-(octyloxy)[1,1'-biphényl]-4-yl]-carbonyl]-L-ornithine]-4-[4-(4-hydroxyphényl)-L-threonine]5-L-serine-échinocandine B(isomère A et isomère B).

On agite quelques minutes à 20°C une solution renfermant 62,5 mg de (S)-(-) dichlorhydrate de diaminopropane, 2,25 ml de méthanol, de la triéthylamine pour obtenir un pH de 6, quelques grains de siliporité activée, et 150 mg du produit du stade précédent. On introduit 6 mg de NaBH₃CN. On agite pendant 15 heures à 20°C et obtient après purification HPLC semi-préparative (éluant : CH₃CN,H₂OTFA(50-50-0,02), 11,5 mg d'isomère A, 13 mg d'isomère B.

### EXEMPLE 2 : Trifluoroacetate de 1-[4-[[(1H-benzimidazol-2-yl)-méthyl)-amino]-N2-[[4"-(pentyloxy) [1,1':4',1"-terphényl]-4-yl]-carbonyl]-L-ornithine]-4-[4-(4-hydroxyphényl)-L-threonine]5-L-serine-échinocandine B(isomère B).

En opérant comme précédemment à partir du nucléus de deoxymulundocandine préparé à la préparation 1 en obtenant comme produit intermédiaire le 1-[(4R,5R)-4,5-dihydroxy-N2-[[4"-(pentyloxy)[1,1' : 4',1''-terphényl]-4-yl]carbonyl]-L-ornithine]-4-[4-(4-hydroxyphényl)-L-thréonine]-5-L-sérine-échinocandine B et le dérivé 4-oxo correspondant, on a obtenu le produit recherché.Isomère A = 7,4 mg, isomère B = 1,2 mg.

### EXEMPLE 3 : Trifluoroacétate de trans 1-[4-[(2-aminocyclo-hexyl)-amino]-N2-[[4"-(pentyloxy) [1,1':4',1"-terphényl]-4-yl]-carbonyl]-L-ornithine]-4-[4-(4-hydroxyphényl)-L-threonine]-5-L-serine-échinocandine B(isomère A).

En opérant comme précédemment, à partir de 166 mg du dérivé 4-oxo préparé ci-dessus et de 78 mg de (1R, 2R)1-2-diaminocyclohexane, on obtient 462 mg de produit brut que l'on chromatographie sur silice en éluant avec le mélange chlorure de méthylène, méthanol, H₂O, acide acétique 86/13/2/1. On obtient 100 mg de produit que l'on purifie par HPLC semi-préparative à nouveau avec le mélange CH₃CN/H₂O/TFA = 50/50/0,1. On obtient 55 mg de l'isomère A, 5,2 mg de l'isomère B.

### EXEMPLE 4 : Trifluoroacétate de 1-[4-[(2(S)-aminopropyl)-amino]-N2-[[4"-(pentyloxy)[1,1':4',1"-terphényl]-4-yl]-carbonyl]-L-ornithine]-4-[4-(4-hydroxyphényl)-L-threonine]-5-L-serine-échinocandine B(isomère A).

En opérant comme précédemment on a obtenu le produit recherché.

### EXEMPLE : Composition pharmaceutique :

On a préparé des comprimés renfermant :
- Produit de l'exemple 3 isomère A 150 mg
- Excipient q.s.p 1 g (Détail de l'excipient : amidon, talc, stéarate de magnésium).

### ETUDE PHARMACOLOGIQUE

### A - Inhibition de la glucane synthase de Candida albicans.

On purifie des membranes de Candida albicans selon le procédé décrit par Tang et al.Antimicrob. Agents Chemother.35, 99-103, 1991. 22,5 µg de protéines membranaires sont incubées dans un mélange de 2Mm de 14C-UDP glucose (activité spécifique = 0,34 mCi./mmol, 50 µg d'α-amylase, 1Mm de dithiotreitol (DTT), 1Mm EDTA, 100Mm NaF, 7µM de GTP-γ-S, 1M de sucrose et 50Mm DE Tris-HCL (pH 7,8) dans un volume de 100µl. Le milieu est incubé à 25°C pendant 1 heure et la réaction terminée par addition de TCA à une concentration finale de 5%. Le mélange réactionnel est transféré sur un filtre de fibre de verre pré-humidifié. Le filtre est lavé, séché et sa radioactivité est comptée.
La mulundocandine est utilisé comme contrôle positif.
Le contrôle du véhicule est effectué avec la même quantité de DMSO 1%. Les résultats obtenus montrent que les produits de l'invention présentent sur ce test une bonne activité en particulier les produits de l'exemple 3 isomère A.

### B - activité sur l'enzyme d'Aspergillus fumigatus.

L'enzyme est préparée selon le procédé de Beaulieu et al.(Antimicrob. Agents Chemother. 38, 937-944, 1994).
Le protocole utilisé est identique au protocole décrit ci-dessus pour l'enzyme de Candida albicans sauf que l'on n'utilise pas de dithiotreitol dans le mélange réactionnel.

Les produits présentent sur ce test une bonne activité.

## Revendications

1. Sous toutes les formes d'isomères possibles ainsi que leurs mélanges, les composés de formule (I) : 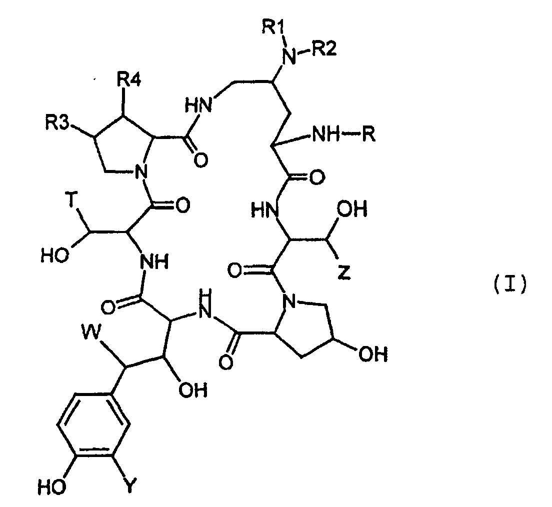 dans lesquels
ou bien R₁ représente un atome d'hydrogène ou un radical méthyle.
R₂ représente un radical cyclohexyle substitué par une amine, un radical CH₂CH₂NHCH₃, un radical CH₂CHCH₃NH₂, un radical 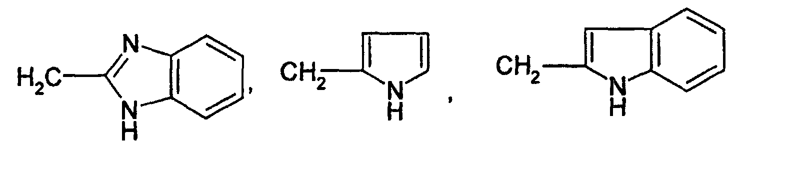 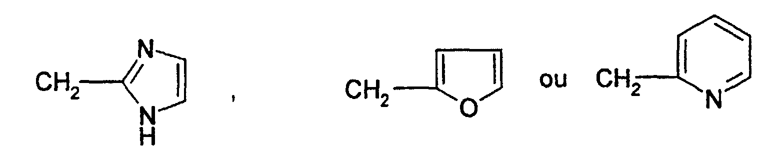 un radical CHCH₃CH₂NH₂, un radical-(CH2)aOH, a représentant un nombre entier compris entre 1 et 8, un radical (CH₂)b-C≡N
b représentant un nombre entier compris entre 1 et 8, un radical CHCH₃C₆H₅, un radical (CH₂)-C(CH₃)₂NHCOCF₃, un radical CHCH₃(CH₂)dOH, d représentant un nombre entier compris entre 1 et 8
ou bien R₁ et R₂ forment avec l'azote qui les porte un cycle à 3, 4 ou 5 carbones éventuellement substitué par une amine
R3 représente un atome d'hydrogène, un radical méthyle ou hydroxyle
R₄ représente un atome d'hydrogène ou un radical hydroxyle
R représente une chaîne linéaire ou ramifiée ou cyclique renfermant jusqu'à 30 atomes de carbone, renfermant éventuellement, un ou plusieurs hétéroatomes, un ou plusieurs hétérocycles ou un radical acyle linaire, ramifié ou cyclique renfermant jusqu'à 30 atomes de carbone renfermant éventuellement un ou plusieurs hétéroatomes et/ou un ou plusieurs hétérocycles,
T représente un atome d'hydrogène, un radical méthyle, un radical CH₂CONH₂, CH₂C≡N, un radical (CH₂)₂NH₂ ou (CH₂)₂Nalc⁺X⁻,
X étant un atome d'halogène et alc un radical alkyle renfermant jusqu'à 8 atomes de carbone,
Y représente un atome d'hydrogène, un radical hydroxyle ou un atome d'halogène ou un radical OSO₃H ou l'un des sels de ce radical,
W représente un atome d'hydrogène ou un radical OH,
Z représente un atome d'hydrogène ou un radical méthyle, ainsi que les sels d'addition avec les acides des produits de formule (I).

2. Les composés de formule (I) définis à la revendication 1 dans lesquels T représente un atome d'hydrogène.

3. Les composés de formule (I) définis à la revendication 1 ou 2 dans lesquels W représente un atome d'hydrogène.

4. Les composés de formule (I) définis à l'une quelconque des revendications 1 à 3, dans lesquels Z représente un radical méthyle.

5. Les composés de formule (I) définis à l'une quelconque des revendications 1 à 4 dans lesquels Y représente un atome d'hydrogène.

6. Les composés de formule (I) définis à l'une quelconque des revendications 1 à 5 dans lesquels R₃ représente un radical méthyle.

7. Les composés de formule définis à l'une quelconque des revendications 1 à 6 dans lesquels R₄ représente un radical hydroxyle.

8. Les composés de formule (I) définis à l'une quelconque des revendications 1 à 7 dans lesquels R représente un radical

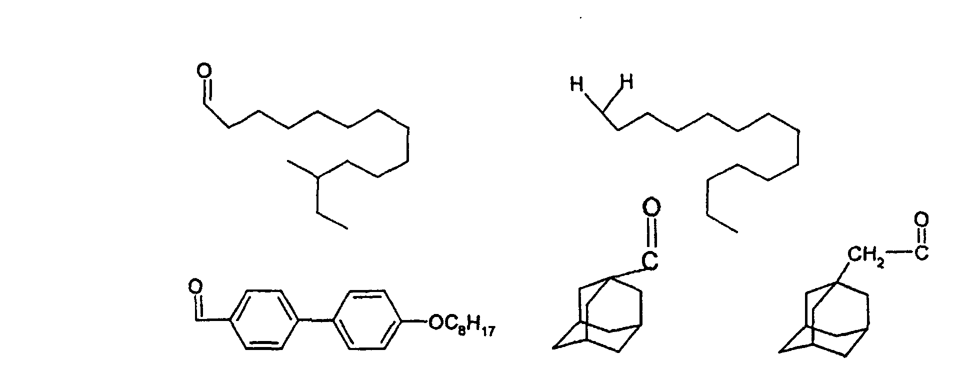

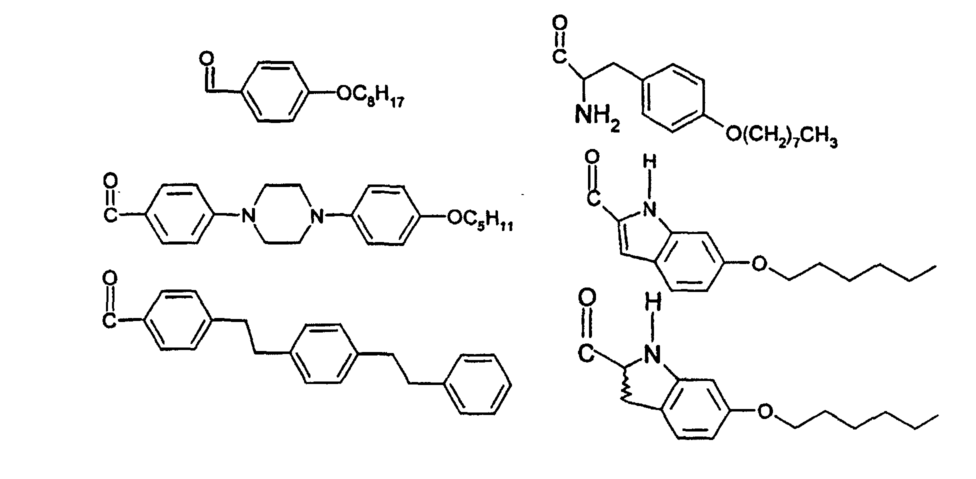

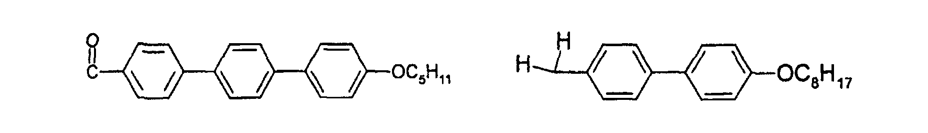

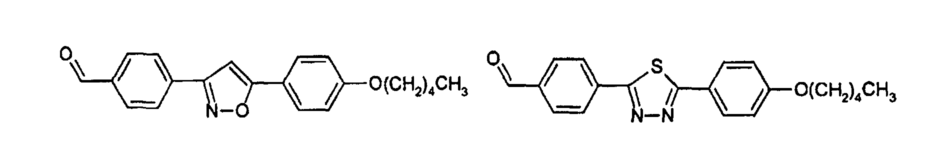

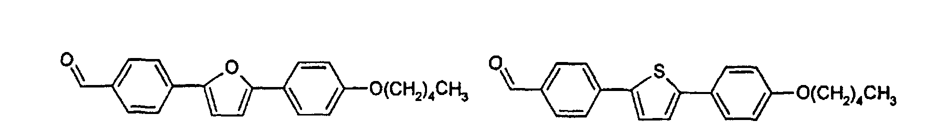

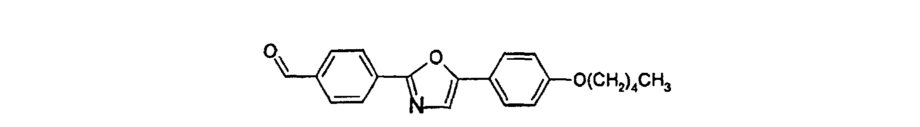

9. Les composés de formule (I) définis à la revendication 8, dans lesquels R représente une chaîne

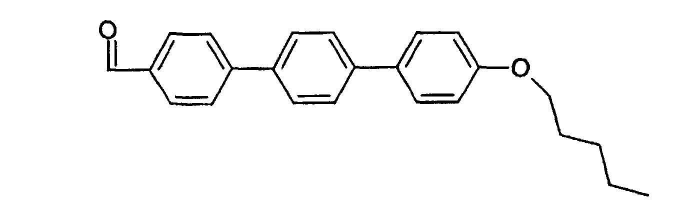

10. Les composés de formule (I) définis à la revendication 8, dans lesquels R représente une chaîne

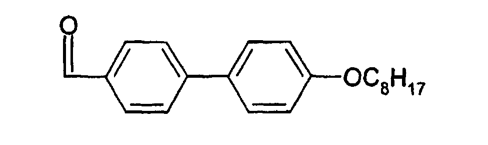

11. Les composés de formule (I) définis à une quelconque des revendications 1 à 10 dans lesquels R₁ est un atome d'hydrogène.

12. Les composés de formule (I) définis à l'une quelconque des revendications 1 à 11 dans lesquels R₂ est un radical

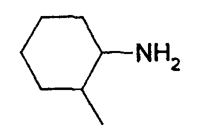

13. Les composés de formule (I) définis à l'une des quelconques revendications 1 à 11 dans lesquels R₂ est un radical 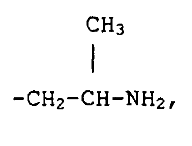 un radical 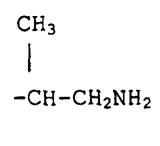 ou un radical

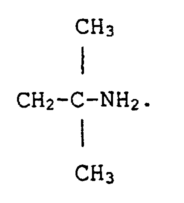

14. Les composés de formule (I) définis à l'une quelconque des revendications 1 à 11 dans lesquels R2 est un radical

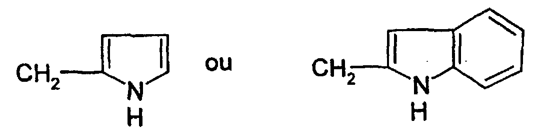

15. Les composés de formule (I) définis à l'une quelconque des revendications 1 à 14 dont les noms suivent :
- Trifluoroacetate de 1-[4-[[(1H-benzimidazol-2-yl)-méthyl)-amino]-N2-[[4"-(pentyloxy)[1,1':4',1"-terphényl]-4-yl]-carbonyl]-L-ornithine]-4-[4-(4-hydroxyphényl)-L-threonine]5-L-serine-échinocandine B(isomère B),
- Trifluoroacétate de trans 1-[4-[(2-aminocyclo-hexyl)-amino]-N2-[[4"-(pentyloxy)[1,1':4',1"-terphényl]-4-yl]-carbonyl]-L-ornithine]-4-[4-(4-hydroxyphényl)-L-threonine]-5-L-serine-échinocandine B(isomère A).

16. Procédé de préparation des composés de formule (I) définis à l'une des quelconques revendications 1 à 15 **caractérisé en ce que** l'on soumet un composé de formule (II) 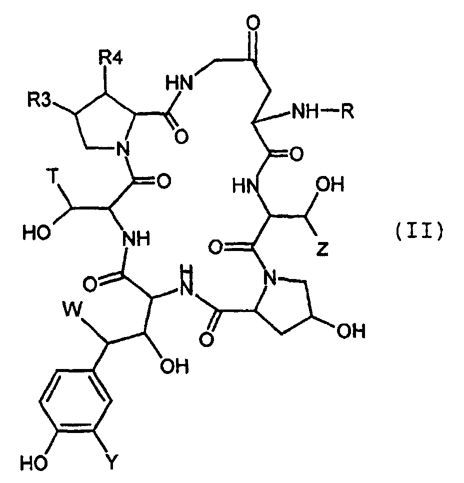 dans laquelle R, R₃, R₄, T, Y, W et Z conservent la signification donnée dans les revendications précédentes, à l'action d'une amine ou d'un dérivé d'amine susceptible d'introduire le radical 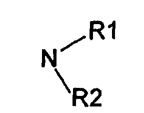 dans lequel R₁ et R₂ conservent la signification donnée dans les revendications précédentes et si désiré à l'action d'un agent de réduction
et/ou d'un agent de fonctionnalisation de l'amine,
et/ou d'un acide pour former le sel du produit obtenu,
et/ou d'un agent de séparation des différents isomères obtenus,
et obtient ainsi le composé de formule (I) recherché.

17. A titre de composés antifongiques, les composés de formule (I) définis à l'une quelconque des revendications 1 à 15, ainsi que leurs sels d'addition avec les acides.

18. Les compositions pharmaceutiques renfermant à titre de médicament au moins un composé de formule (I) défini à l'une quelconque des revendications 1 à 15, ainsi que leurs sels d'addition avec les acides pharmaceutiquement acceptables.

## Claims

1. In all possible isomer forms as well as their mixtures, the compounds of formula (I): 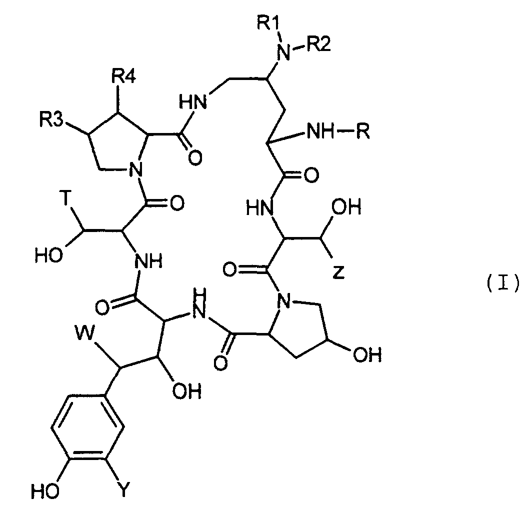 in which
either R₁ represents a hydrogen atom or a methyl radical.
R₂ represents a cyclohexyl radical substituted by an amine, a CH₂CH₂NHCH₃ radical, a CH₂CHCH₃NH₂ radical, a 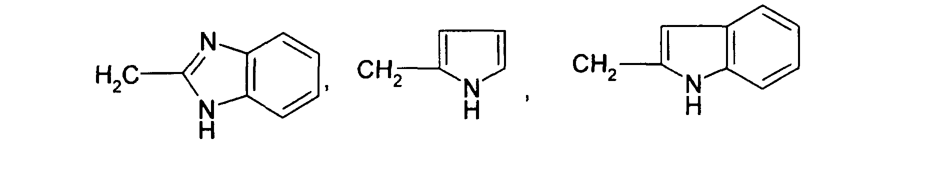 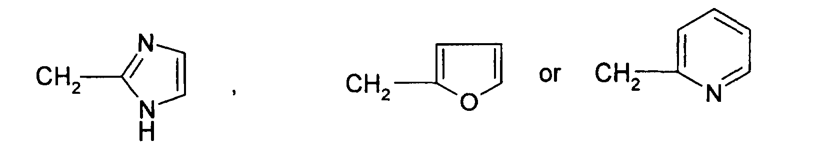 radical, a CHCH₃CH₂NH₂ radical, a -(CH2)aOH radical, a representing an integer comprised between 1 and 8, a (CH₂)b-C≡N radical
b representing an integer comprised between 1 and 8, a CHCH₃C₆H₅ radical, a (CH₂)-C(CH₃)₂NHCOCF₃ radical, a CHCH₃(CH₂)dOH radical, d representing an integer comprised between 1 and 8
or R₁ and R₂ together with the nitrogen which carries them form a ring with 3, 4 or 5 carbons optionally substituted by an amine
R3 represents a hydrogen atom, a methyl or hydroxyl radical
R₄ represents a hydrogen atom or a hydroxyl radical
R represents a linear or branched or cyclic chain containing up to 30 carbon atoms, optionally containing one or more heteroatoms, one or more heterocycles or a linear, branched or cyclic acyl radical containing up to 30 carbon atoms optionally containing one or more heteroatoms and/or one or more heterocycles,
T represents a hydrogen atom, a methyl radical, a CH₂CONH₂, CH₂C≡N radical, a (CH₂)₂NH₂ or (CH₂)₂Nalc⁺X⁻ radical, X being a halogen atom and alk an alkyl radical containing up to 8 carbon atoms,
Y represents a hydrogen atom, a hydroxyl radical or a halogen atom or an OSO₃H radical or one of the salts of this radical,
W represents a hydrogen atom or an OH radical,
Z represents a hydrogen atom or a methyl radical,
as well as the addition salts with acids of the products of formula (I).

2. The compounds of formula (I) defined in claim 1 in which T represents a hydrogen atom.

3. The compounds of formula (I) defined in claim 1 or 2 in which W represents a hydrogen atom.

4. The compounds of formula (I) defined in any one of claims 1 to 3, in which Z represents a methyl radical.

5. The compounds of formula (I) defined in any one of claims 1 to 4 in which Y represents a hydrogen atom.

6. The compounds of formula (I) defined in any one of claims 1 to 5 in which R₃ represents a methyl radical.

7. The compounds of formula defined in any one of claims 1 to 6 in which R₄ represents a hydroxyl radical.

8. The compounds of formula (I) defined in any one of claims 1 to 7 in which R represents a 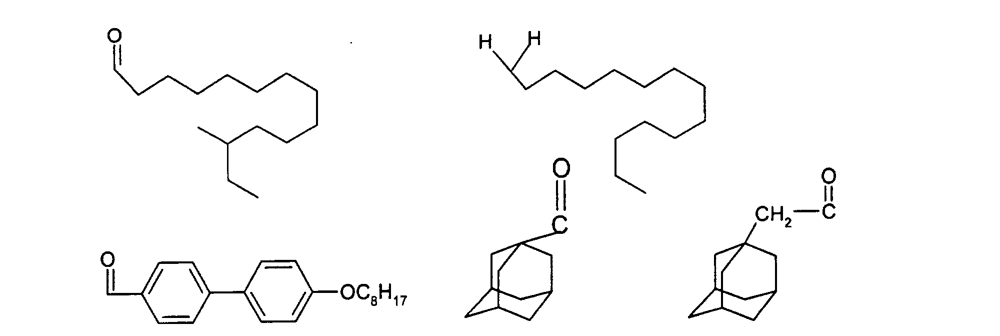 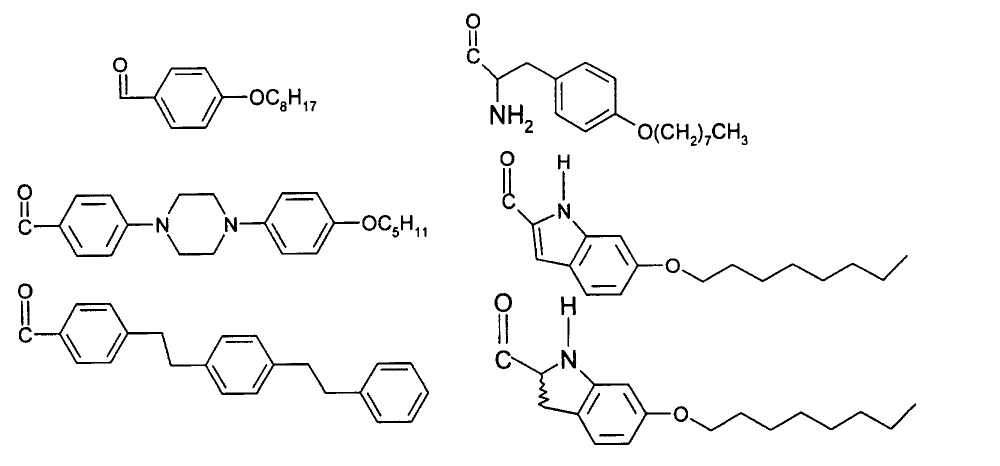 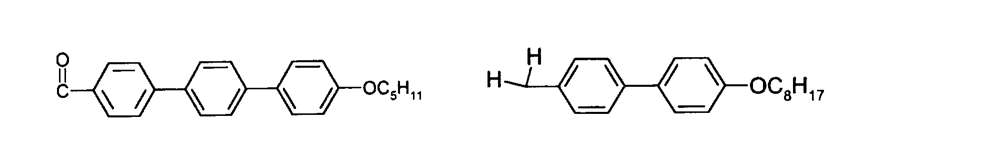 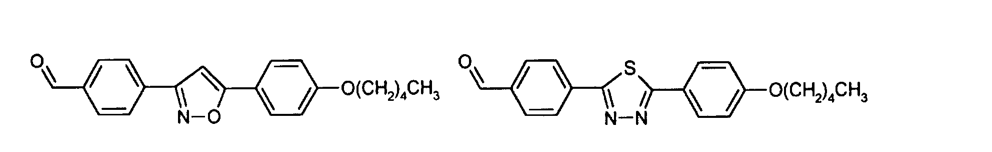 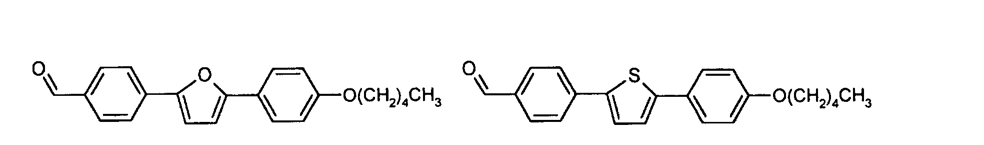 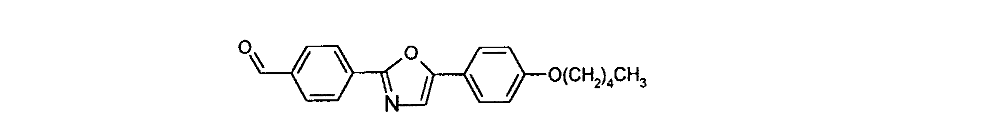 radical.

9. The compounds of formula (I) defined in claim 8, in which R represents a 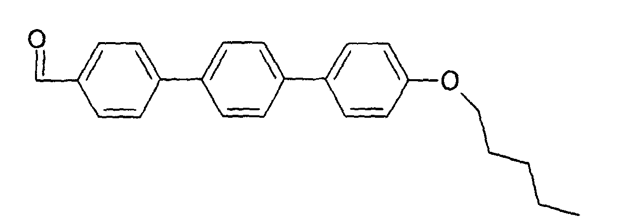 chain.

10. The compounds of formula (I) defined in claim 8, in which R represents a 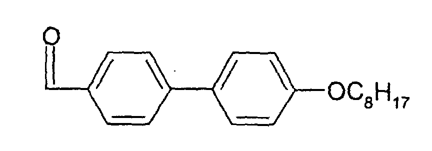 chain.

11. The compounds of formula (I) defined in any one of claims 1 to 10 in which R₁ is a hydrogen atom.

12. The compounds of formula (I) defined in any one of claims 1 to 11 in which R₂ is a 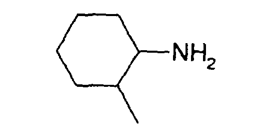 radical.

13. The compounds of formula (I) defined in any one of claims 1 to 11 in which R₂ is a 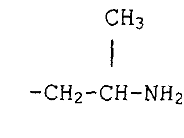 radical, a 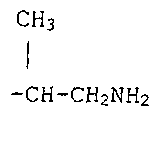 radical or a 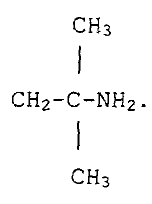 radical.

14. The compounds of formula (I) defined in any one of claims 1 to 11 in which R2 is a 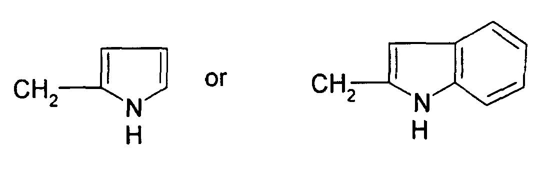 radical.

15. The compounds of formula (I) defined in any one of claims 1 to 14 the names of which follow:
- 1-[4-[[(1H-benzimidazol-2-yl)-methyl)-amino]-N2-[[4"-(pentyloxy)[1,1':4',1"-terphenyl]-4-yl]-carbonyl]-L-ornithine]-4-[4-(4-hydroxyphenyl)-L-threonine]5-L-serine-echinocandine B trifluoroacetate (B isomer),
- trans 1-[4-[(2-aminocyclo-hexyl)-amino]-N2-[[4"-(pentyloxy)[1,1':4',1"-terphenyl]-4-yl]-carbonyl]-L-ornithine]-4-[4-(4-hydroxyphenyl)-L-threonine]-5-L-serine-echinocandine B trifluoroacetate (A isomer).

16. Process for the preparation of the compounds of formula (I) defined in any one of claims 1 to 15, **characterized in that** a compound of formula (II) 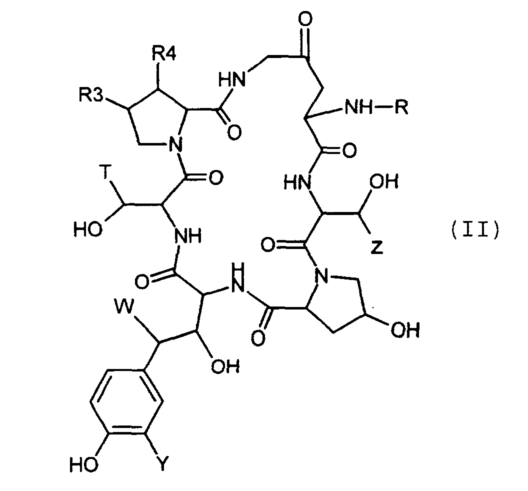 in which R, R₃, R₄, T, Y, W and Z retain the meaning given in the preceding claims, is subjected to the action of an amine or amine derivative capable of introducing the 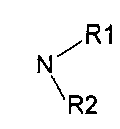 radical in which R₁ and R₂ retain the meaning given in the preceding claims and if desired to the action of a reducing agent
and/or an amine functionalization agent,
and/or an acid in order to form the salt of the product obtained,
and/or a separation agent of the different isomers obtained, and the sought compound of formula (I) is obtained in this way.

17. As antifungal compounds, the compounds of formula (I) defined in any one of claims 1 to 15, as well as their addition salts with acids.

18. Pharmaceutical compositions containing as a medicament at least one compound of formula (I) defined in any one of claims 1 to 15, as well as their addition salts with pharmaceutically acceptable acids.

## Patentansprüche

1. In allen möglichen isomeren Formen sowie ihren Mischungen die Verbindungen der Formel (I) 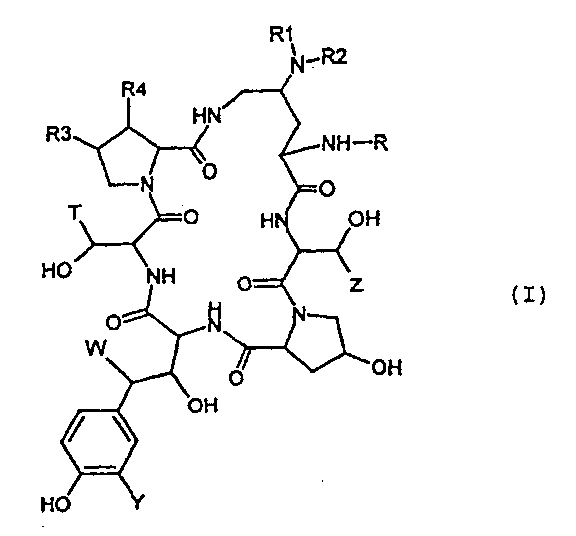 in der
entweder R₁ ein Wasserstoffatom oder einen Rest Methyl darstellt,
R₂ einen Rest Cyclohexyl, substituiert durch ein Amin, einen Rest CH₂CH₂NHCH₃, einen Rest CH₂CHCH₃NH₂, einen Rest 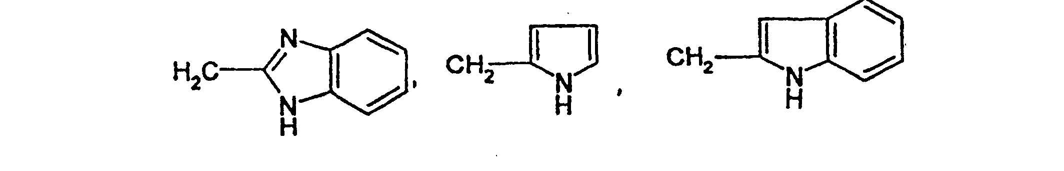 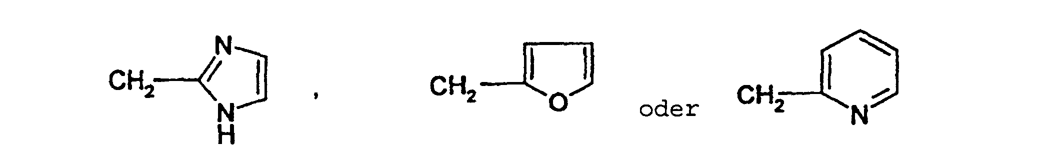 einen Rest CHCH₃CH₂NH₂, einen Rest -(CH₂)aOH, worin a eine ganze Zahl zwischen 1 und 8 ist, einen Rest (CH₂)b-C≡N, worin b eine ganze Zahl zwischen 1 und 8 ist, einen Rest CHCH₃C₆H₅, einen Rest (CH₂)-C(CH₃)₂NHCOCF₃, einen Rest CHCH₃(CH₂)dOH, worin d eine ganze Zahl zwischen 1 und 8 ist, bedeutet,
oder R₁ und R₂ zusammen mit dem Stickstoff, der sie trägt, einen Ring mit 3, 4 oder 5 Kohlenstoffen bilden, gegebenenfalls substituiert durch ein Amin,
R₃ ein Wasserstoffatom, einen Rest Methyl oder Hydroxyl darstellt,
R₄ ein Wasserstoffatom oder ein Rest Hydroxyl ist,
R eine lineare, verzweigte oder cyclische Kette mit bis zu 30 Kohlenstoffatomen darstellt, die gegebenenfalls ein oder mehrere Heteroatome, einen oder mehrere Heterocyclen oder einen linearen, verzweigten oder cyclischen Rest Acyl mit bis zu 30 Kohlenstoffatomen umfaßt, der seinerseits gegebenenfalls ein oder mehrere Heteroatome und/oder einen oder mehrere Heterocyclen umfaßt,
T ein Wasserstoffatom, einen Rest Methyl, einen Rest CH₂CONH₂, CH₂C≡N, einen Rest (CH₂)₂NH₂ oder (CH₂)₂Nalc⁺X⁻ bedeutet,
X ein Halogenatom ist und alc einen Rest Alkyl mit bis zu 8 Kohlenstoffatomen darstellt,
Y ein Wasserstoffatom, einen Rest Hydroxyl oder ein Halogenatom oder einen Rest OSO₃H oder eines der Salze von diesem Rest bedeutet,
W ein Wasserstoffatom oder ein Rest OH darstellt,
Z ein Wasserstoffatom oder ein Rest Methyl darstellt,
sowie die Additionssalze der Produkte der Formel (I) mit Säuren.

2. Verbindungen der Formel (I) wie in Anspruch 1 definiert, worin T ein Wasserstoffatom darstellt.

3. Verbindungen der Formel (I) wie in Anspruch 1 oder 2 definiert, worin W ein Wasserstoffatom darstellt.

4. Verbindungen der Formel (I) wie in irgendeinem der Ansprüche 1 bis 3 definiert, worin Z einen Rest Methyl darstellt.

5. Verbindungen der Formel (I) wie in irgendeinem der Ansprüche 1 bis 4 definiert, worin Y ein Wasserstoffatom darstellt.

6. Verbindungen der Formel (I) wie in irgendeinem der Ansprüche 1 bis 5 definiert, worin R₃ einen Rest Methyl darstellt.

7. Verbindungen der Formel (I) wie in irgendeinem der Ansprüche 1 bis 6 definiert, worin R₄ einen Rest Hydroxyl darstellt.

8. Verbindungen der Formel (I) wie in irgendeinem der Ansprüche 1 bis 7 definiert, worin R einen der folgenden Reste darstellt:

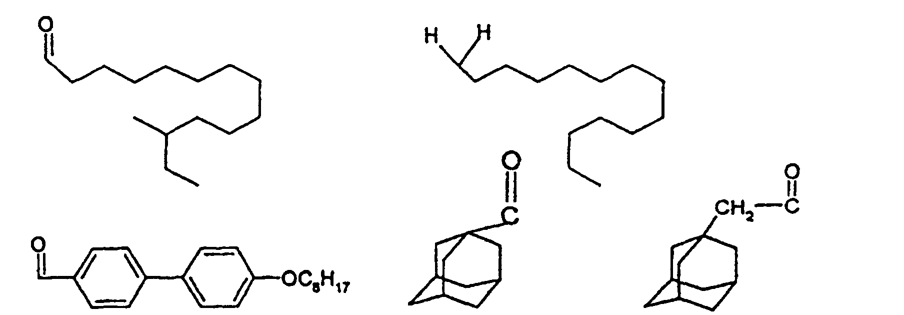

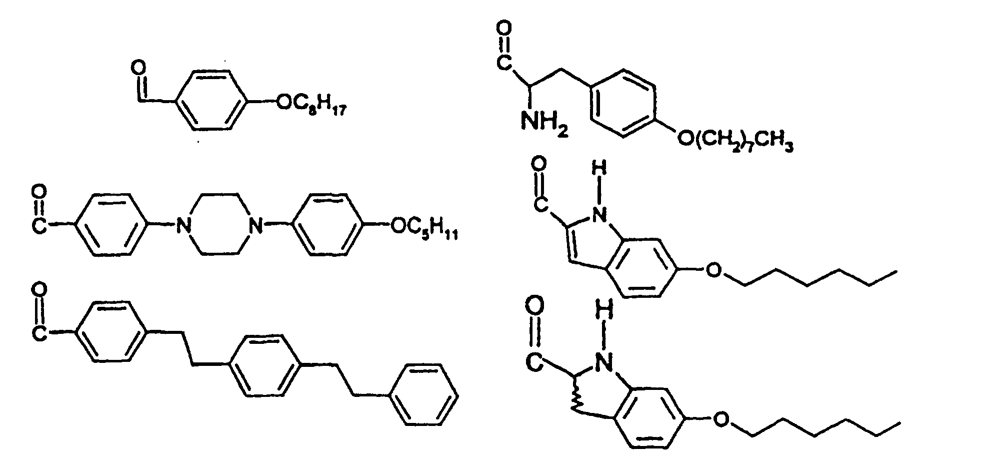

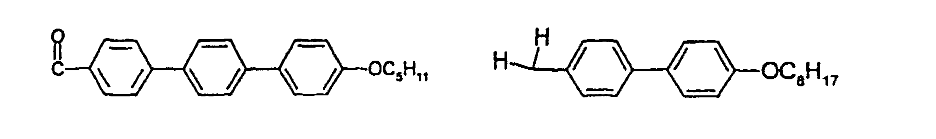

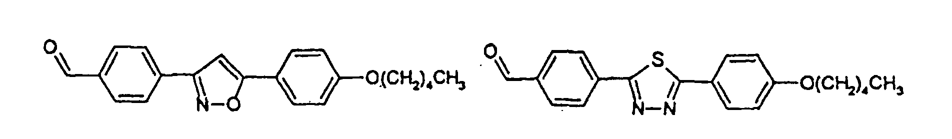

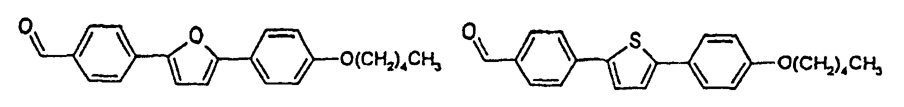

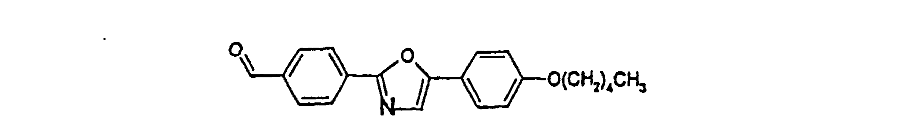

9. Verbindungen der Formel (I) wie in Anspruch 8 definiert, worin R eine Kette 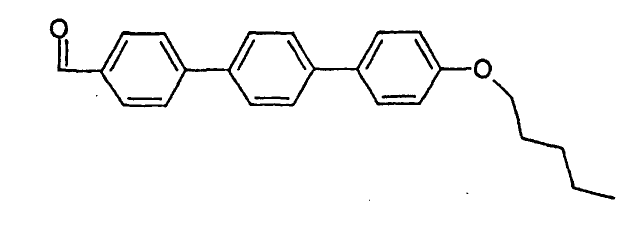 darstellt.

10. Verbindungen der Formel (I) wie in Anspruch 8 definiert, worin R eine Kette 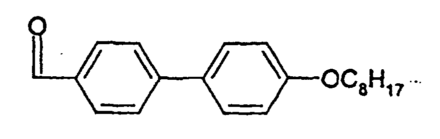 darstellt.

11. Verbindungen der Formel (I) wie in irgendeinem der Ansprüche 1 bis 10 definiert, worin R₁ ein Wasserstoffatom ist.

12. Verbindungen der Formel (I) wie in irgendeinem der Ansprüche 1 bis 11 definiert, worin R₂ einen Rest 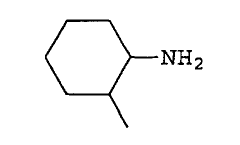 darstellt.

13. Verbindungen der Formel (I) wie in irgendeinem der Ansprüche 1 bis 11 definiert, worin R₂ einen Rest 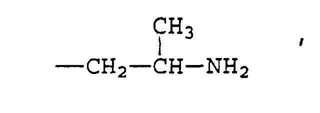 einen Rest 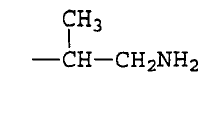 oder einen Rest 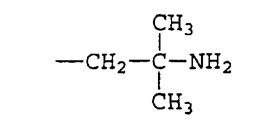 darstellt.

14. Verbindungen der Formel (I) wie in irgendeinem der Ansprüche 1 bis 11 definiert, worin R₂ einen Rest 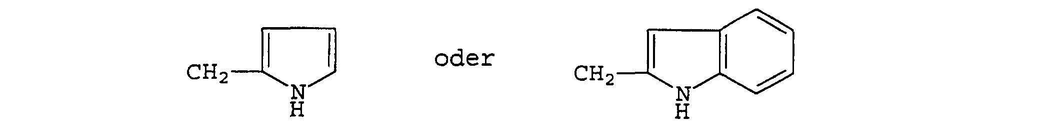 darstellt.

15. Verbindungen der Formel (I) wie in irgendeinem der Ansprüche 1 bis 14 definiert, mit den folgenden Namen:
- Trifluoracetat von 1-{4-[[(1H-Benzimidazol-2-yl)-methyl]-amino]-N2-[[4"-(pentyloxy)-[1,1':4',1"-terphenyl]-4-yl-]-carbonyl]-L-ornithin}-4-[4-(4-hydroxyphenyl)-L-threonin]-5-L-serinechinocandin B (Isomer B),
- Trifluoracetat von trans-1-{4-[(2-Aminocyclohexyl)-amino]-N2-[[4"-(pentyloxy)-[1,1':4',1"-terphenyl]-4-yl-]-carbonyl]-L-ornithin}-4-[4-(4-hydroxyphenyl)-L-threonin]-5-L-serin-echinocandin B (Isomer A).

16. Verfahren zur Herstellung der Verbindungen der Formel (I) wie in irgendeinem der Ansprüche 1 bis 15 definiert, **dadurch gekennzeichnet, daß** man eine Verbindung der Formel (II) 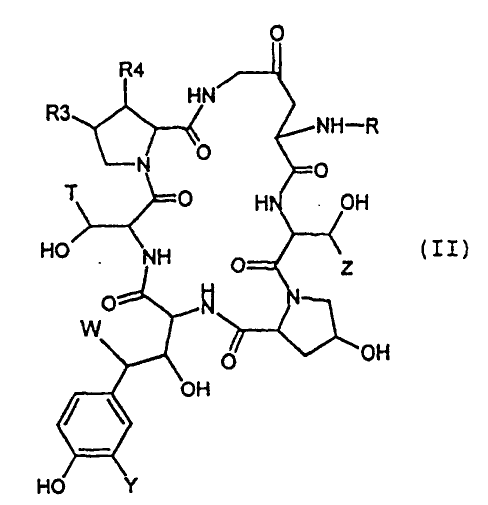 in der R, R₃, R₄, T, Y, W und Z ihre in den vorstehenden Ansprüchen angegebene Bedeutung beibehalten, der Einwirkung eines Amins oder eines Derivates eines Amins unterzieht, das geeignet ist, den Rest 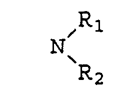 einzubringen, worin R₁ und R₂ ihre in den vorstehenden Ansprüchen angegebene Bedeutung beibehalten, und wenn erwünscht, der Einwirkung eines Reduktionsmittels, und/oder eines Mittels zur Funktionalisierung des Amis, und/oder einer Säure, um das Salz des erhaltenen Produktes zu bilden, und/oder eines Mittels zur Trennung der verschiedenen erhaltenen Isomeren unterzieht, und auf diese Weise die gesuchte Verbindung der Formel (I) erhält.

17. Als fungizide Verbindungen die Verbindungen der Formel (I) wie in irgendeinem der Ansprüche 1 bis 15 definiert, sowie ihre Additionssalze mit Säuren.

18. Pharmazeutische Zusammensetzungen, umfassend als Arzneimittel mindestens eine Verbindung der Formel (I) wie in irgendeinem der Ansprüche 1 bis 15 definiert, sowie ihre Additionssalze mit pharmazeutisch akzeptablen Säuren.
